# EUROPEAN PATENT APPLICATION

(11) **EP 2 752 813 A1**
(43) Date of publication of application: **09.07.2014**
(21) Application number: 12828609.3
(22) Date of filing: 07.06.2012
(51) Int. Cl.: G06Q 50/22, G06Q 10/00

(54) **TRIAGE TAG MANAGEMENT SYSTEM AND SMARTPHONE FOR SAME, AND TRIAGE TAG MANAGEMENT METHOD**

(30) Priority: 31.08.2011 JP 2011189327
(71) Applicant: Tokyo Electronic Systems Corporation, Kanagawa 212-0001 (JP)
(72) Inventor: NAKAMORI, Yasushi, Osaka-shi Osaka 588-0056 (JP); TANAKA, Hideshige, Kawasaki-shi Kanagawa 212-0001 (JP); ITO, Akira, Kawasaki-shi Kanagawa 212-0001 (JP); NAKAMARU, Tomoko, Kawasaki-shi Kanagawa 212-0001 (JP); KACHI, Kyosuke, Kawasaki-shi Kanagawa 212-0001 (JP); HOMMA, Yoshio, Kawasaki-shi Kanagawa 212-0001 (JP)
(74) Representative: HOFFMANN EITLE
(86) International application number: PCT/JP2012/003741
(87) International publication number: WO 2013/031067

(57) **Abstract**

An example of a triage tag management includes a triage tag attached to a wounded person, a smartphone that receives input of symptom data of the wounded person, determines a symptom level of the wounded person, and transmits data of the symptom level to the triage tag, and a wounded person information database that receives the symptom level data of the wounded person from the smartphone over a network and stores the symptom level data therein for each wounded person attached with the triage tag, the smartphone including a temporary storage section that temporarily stores the symptom level data of the wounded person when the symptom level data cannot be transmitted to the wounded person information database.

## Description

### TECHNICAL FIELD

The present invention relates to a triage tag management system for providing appropriate care for wounded persons, and a smartphone and a triage tag management method therefor.

### BACKGROUND ART

A triage tag management system is known as a system for urgently providing appropriate care for wounded persons when a large-scale disaster such as an earthquake or a typhoon occurs. In such a system, a triage tag is attached to each wounded person so as to grasp a damage situation, and adequate care is provided based on the information. The triage tag is an identification tag indicating treatment priority for the wounded person to which the tag is attached.

There is known a systemthat sends the damage situation of each wounded person acquired based on the triage tag using a mobile terminal to a database connected to a network and thereby manages conditions of all wounded persons.

In this conventional system, the mobile terminal acquires wounded person information from the triage tag and sends the information to the database over the network so as to update information such as symptoms of the wounded persons. However, although an adequate response can be made provided that the mobile terminal and database are always stably connected to each other, it sometimes happens that network connection fails. In particular, when such a large-scale disaster occurs, the network often loses its stability. In addition, unless symptom-based treatment for the wounded person is made as quickly as possible, bad results may be caused.

### CITATION LIST

### PATENT LITERATURE

PLT 1: Japanese Patent Application Laid-Open No. 2004-240797

### SUMMARY OF THE INVENTION

### TECHNICAL PROBLEM

An object of the present invention is to provide a triage tag management system capable of grasping the latest information such as symptoms of wounded persons to thereby adequately make an appropriate response even under a condition that the network connection state becomes unstable as described above, and a smartphone and a triage tag management method therefor.

### SOLUTION TO PROBLEM

According to an aspect of the invention, there is provided a triage tag management system including a triage tag attached to a wounded person, a smartphone that receives input of symptom data of the wounded person, determines a symptom level of the wounded person, and transmits data of the symptom level to the triage tag, and a wounded person information database that receives the symptom level data of the wounded person from the smartphone over a network and stores the symptom level data therein for each wounded person attached with the triage tag, the smartphone including a temporary storage section that temporarily stores the symptom level data of the wounded person when the symptom level data cannot be transmitted to the wounded person information database.

### BRIEF DESCRIPTION OF THE DRAWINGS

FIG. 1 is a view illustrating the entire system according to an embodiment of the present invention.
FIG. 2 is a view illustrating a configuration example of a smartphone in an embodiment.
FIG. 3 is a view illustrating a configuration example of a triage tag in an embodiment.
FIG. 4 is a view illustrating a configuration example of a triage support server in an embodiment.
FIG. 5 is a flowchart illustrating operation in an embodiment with a focus on the smartphone.
FIG. 6 is a flowchart illustrating operation when a symptom level is transmitted to the triage tag and triage support server in FIG. 5.
FIG. 7 is a flowchart illustrating operation in an embodiment when a smartphone that determines a symptom level is replaced by another smartphone.
FIG. 8 is an example of a display screen of the smartphone in an embodiment.
FIG. 9 is a view illustrating an example of a screen on which symptoms of a wounded person are input based on a START method in an embodiment.
FIG. 10 is a view illustrating an example of a screen on which symptoms of a wounded person are input based on a PAT method in an embodiment.
FIG. 11 is a view illustrating an example of a screen on which symptoms of a wounded person are input based on a vital sign in an embodiment.
FIG. 12 is a view illustrating another example of the triage tag in an embodiment.
FIG. 13 is a view illustrating still another example of the triage tag in an embodiment.

### DESCRIPTION OF EMBODIMENTS

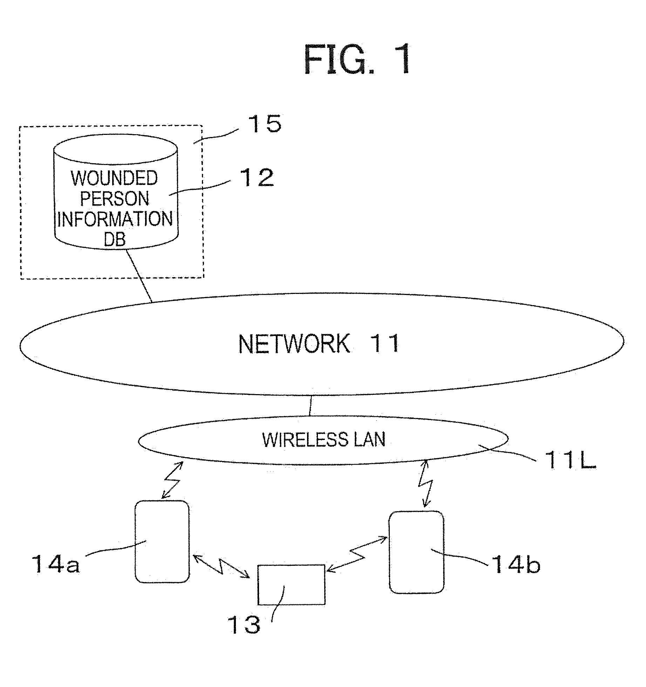

An embodiment of the present invention will be described below with reference to the drawings. FIG. 1 illustrates a configuration example of a triage tag management system according to an embodiment.

The triage management system includes a wounded person information database (DB) 12 connected to a network such as the Internet, smartphones 14a and 14b (sometimes collectively referred to as "smartphone 14" as needed) that read out information of a wounded person from a triage tag 13 attached to the wounded person and write information on a determination result with respect to the wounded person in the triage tag 13 attached thereto, and further write information on the wounded person in the wounded person information DB 12 and read out the wounded person information from the wounded person information database 12. The wounded person information DB 12 is provided in a triage support server 15.

In FIG. 1, one triage tag 13 and two smartphones 12 are provided but they are not limited to this, and generally larger numbers of triage tags and smartphones are used.

The triage tag 13 is an electronic tag which is attached with a unique individual ID and stores personal information, such as a photo of a wounded person, and a medical condition of the wounded person and additionally stores information such as treatment priority after emergency determination made on site. The triage tag 13 is attached to the wounded person on site by a cord or a rubber band.

The triage tag 13 incorporates therein an IC tag provided with an antenna and thus can communicate with an external device by wireless. The IC tag stores a unique and non-rewritable or rewritable tag ID for identifying the IC tag, age, gender, blood type, personal medical history, address, phone number, names of family members, phone numbers of family members, contact information on family members, relationship to a contact person, and others of the wounded person.

The information stored in the IC tag of the triage tag 13 are written by the smartphones 14a and 14b, excluding the tag ID. The following describes the smartphone used in the triage tag management system. The smartphone is a general-purpose smartphone and has a touch panel function. That is, a display is made on a screen of the smartphone, and a part of the screen is touched for input operation. When the triage tag management system is used, a triage icon displayed on the screen of the smartphone is tapped. Then, a predetermined triage program previously installed is activated according to the tapping. Of course, it is possible to use not only the smartphone using the triage program, but also a pre-install type dedicated smartphone (mobile terminal).

Under control of the triage program, reading and writing of information from/into the triage tag are performed directly or through the IC chip provided near the smartphone.

In general, when a large disaster occurs, a disaster medical assistance team is urgently organized. The disaster medical assistance team (DMAT) is composed of doctors, nurses, other medical care staffs and clerical staffs. The members of the DMAT each have the smartphone in which the above-mentioned triage program has been installed. The member carrying the smartphone evaluates symptoms of the wounded person and performs selective input operation, whereby symptom levels (four levels from 0 to 3) of the disaster wounded person based on triage information according to a START method, triage information according to a PAT method, and vital sign information are automatically determined sequentially in this order.

The START is initial letters of "Simple Triage And Rapid Treatment" and is a determination method applied in a case where the number of wounded persons is significantly larger than the number of rescuers, in which determination criteria are made as objective and simple as possible. The PTA method is initial letters of "Physiological and Anatomical Triage" and is originally a determination method for children, which, however, is now used for adults. The vital sign is, literally, a determination method for checking vital signs, i.e., signs of life.

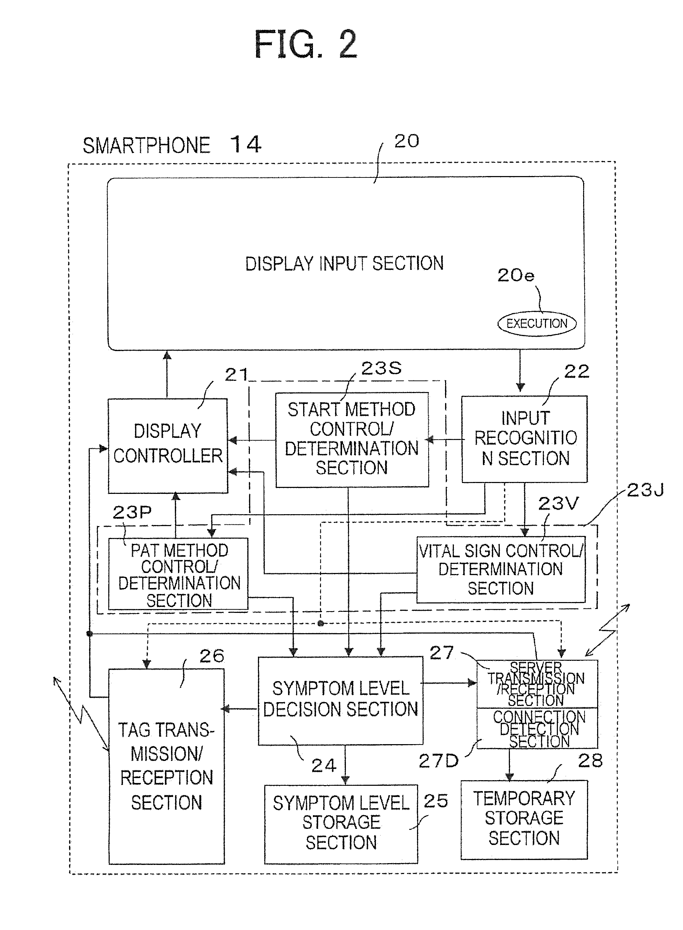

FIG. 2 illustrates a configuration example of the smartphone when the triage program is activated. The smartphone 14 illustrated in FIG. 2 includes a display input section 20 that displays an image on a screen thereof and performs input processing upon a touch operation, etc., on a part of the displayed image, a display controller 21 that controls the image to be displayed on the screen of the display input section 20, an input recognition section 22 that recognizes the input made on the screen of the display input section 20, a START method control/determination section 23S that performs control so as to display a list of questions based on the START method and inputs thereto symptoms of the wounded person to determine his or her conditions, a PAT method control/determination section 23P that performs control so as to display a list of questions based on the PAT method and inputs thereto symptoms of the wounded person to determine his or her conditions, a vital sign control/determination section 23V that performs control so as to display a list of questions on the vital sign and inputs thereto corresponding symptoms to determine the conditions of the wounded person, a symptom level decision section 24 that decides the symptom level of the wounded person based on evaluation on the symptoms made by the START method, PAT method, and vital sign, a symptom level storage section 25 that stores a result decided by the symptom level decision section 24 together with an evaluation time, a tag transmission/reception section 26 that transmits by wireless a final evaluation to the triage tag 13 and receives information from the triage tag 13, a server transmission/reception section 27 that transmits the wounded person symptom level acquired by the symptom level decision section 24 to the triage support server 15 and receives information from the triage support server 15, and a temporary storage section 28 that temporarily stores information when the final evaluation information, etc., cannot be transmitted to the triage support server 15 due to loss of network connection.

The START method control/determination section 23S, PAT method control/determination section 23P, and vital sign control/determination section 23V constitute a symptom determination section 23J that determines the symptom level of the wounded person based on the respective determination methods.

The display input section 20 is a section in which usual display and input processing in the smartphone 4 are performed. When a user performs input operation in response to displayed questions and then touches a displayed execution icon 20e, the input data is recognized by the input recognition section 22 and is then transmitted to the triage tag 13 and triage support server 15 through the tag transmission/reception section 26 and server transmission/reception section 27, respectively.

First, the unique ID of the triage tag 13 is transmitted from the triage tag 13 and received by the tag transmission/reception section 26. Then, the unique ID is passed, through the display controller 21, display input section 20, and input recognition section 22, to the server transmission/reception section 27 and is transmitted therefrom to the triage support server 15. Alternatively, the unique ID may be passed directly to the server transmission/reception section 27 for transmission to the triage support server 15.

A screen for inputting personal information is displayed first on the display input section 20, and the personal information is recognized by the input recognition section 22 based on the input operation. As denoted by dashed line, the input personal information is transmitted directly to the triage tag 13 from the tag transmission/reception section 26 and, at the same time, transmitted to the triage support server 15 from the server transmission/reception section 27 through a wireless LAN 11L and network 11. Data to be transmitted includes data of the determined symptom level and determination time (including year, month, and day) thereof and other input numerical data. Whether the data can be transmitted from the smartphone 14 to the network 11 is determined by a connection detection section 27D provided in the server transmission/reception section 27.

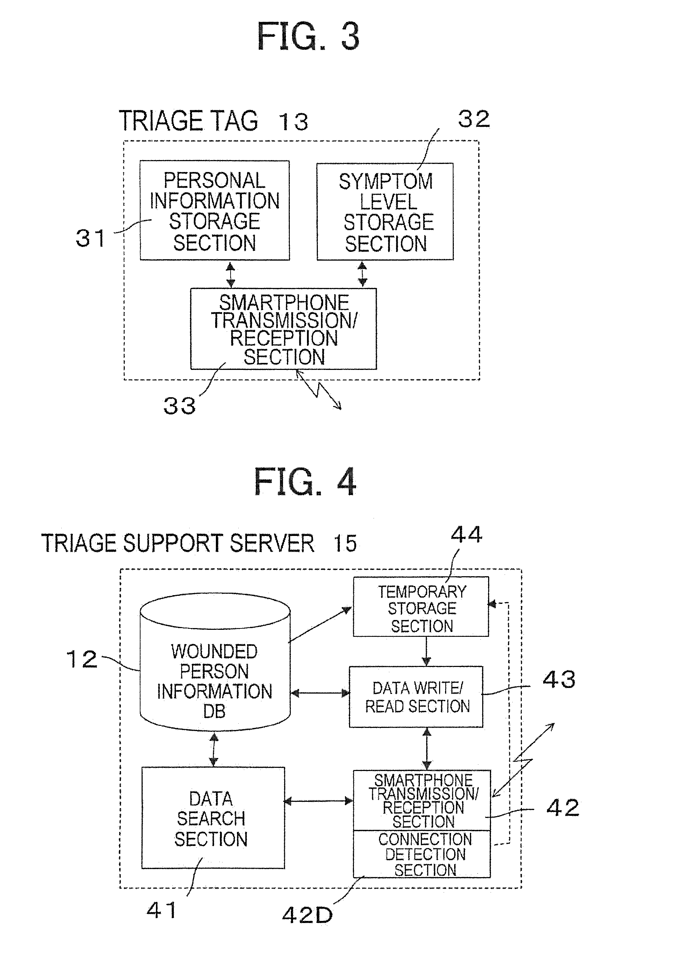

FIG. 3 illustrates a configuration example of the triage tag 13. The triage tag 13 includes a personal information storage section 31 that stores personal basic information, a symptom level storage section 32 that stores the symptom level and determination time thereof transmitted from the smartphone 14 and data of information input from the smartphone 14, and a smartphone transmission/reception section 33 that performs data transmission/reception to/from the smartphone 14.

The data such as the symptom level transmitted from the smartphone 14 through the network 11 is received by the smartphone transmission/reception section 33 and is stored in the triage tag 13.

FIG. 4 illustrates a configuration example of the triage support server 15. The triage support server 15 includes the above-described wounded person information DB 12, a data search section 41 that searches the wounded person information DB 12 based on, e.g., a wounded person name, number, or the like, a smartphone transmission/reception section 42 that performs data transmission/reception to/from the smartphone 14, a connection detection section 42D that is provided in the smartphone transmission/reception section 42 and detects connection to the network 11, a data write/read section 43 that writes/reads data in/from the wounded person information DB 12, and a temporary storage section 44 that temporarily stores data to be transmitted when the connection detection section 42D detects that the data cannot be sent to the network 11.

The data such as the symptom level transmitted from the smartphone 14 is normally received by the smartphone transmission/reception section 42 and is then stored in the wounded person information DB 12 through the data write/read section 43.

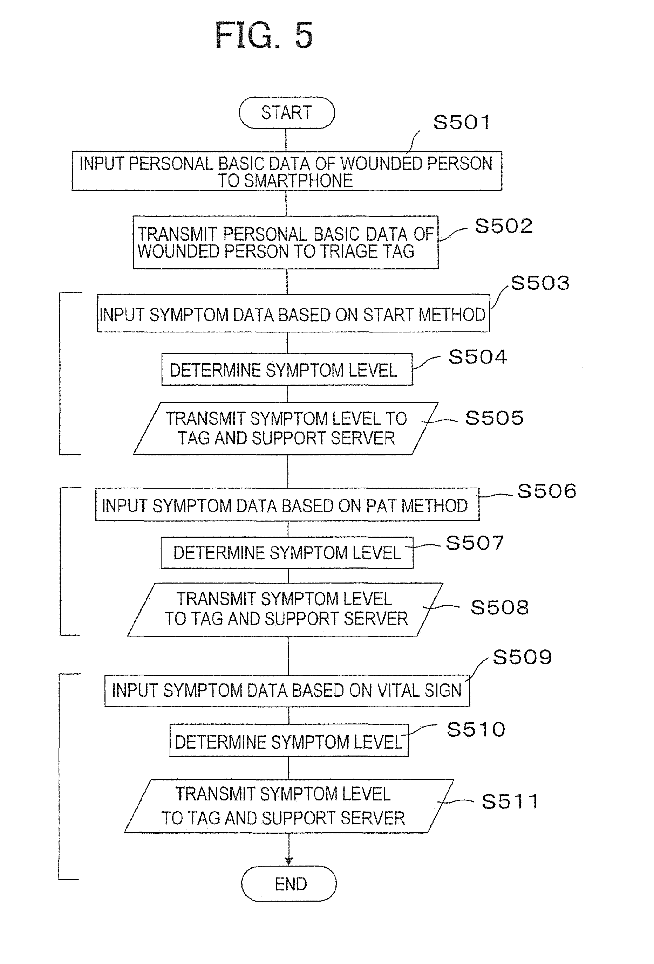

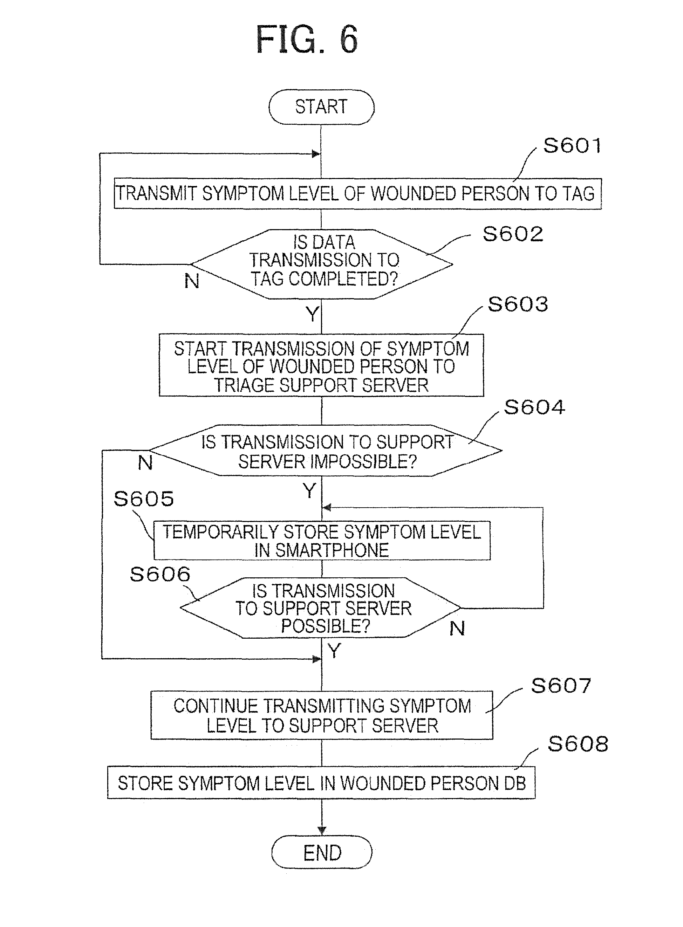

The following describes operation of the present embodiment along flowcharts illustrated in FIGS. 5 and 6. FIG. 5 is a flowchart illustrating input operation of the personal data of the wounded person to the smartphone 14, input operation of symptom data to the smartphone 14 based on the three determination methods of START method, PAT method, and vital sign, and determination result with respect to the input data. FIG. 6 is a flowchart illustrating transmission of the data based on each of the determination methods from the smartphone to the triage tag performed in each of steps S505, S508, and S512 and subsequent data transmission to the triage support server.

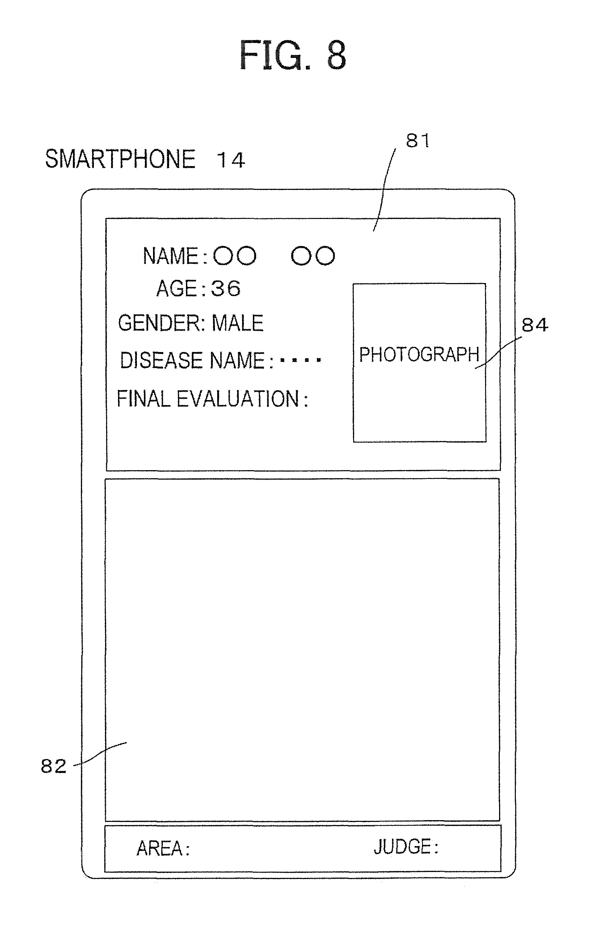

In step S501 illustrated in FIG. 5, a doctor of a given wounded person inputs personal basic data of the wounded person to his or her own smartphone 14a. FIG. 8 illustrates an example of screen display of the input personal basic data.

First, a basic screen to be displayed first on the smartphone 14 (sometimes separately referred to as "smartphone 14a and smartphone 14b" as needed) and a screen to which the personal information is input will be described. The display screen is divided vertically into two parts: an upper basic information display section 81 and a lower condition input/determination display section 82. The personal basic data is fixedly displayed in the upper basic information display section 81. The lower condition input/determination display section 82 can be scrolled downward by downward finger dragging, in which questions to be described later of the three determination methods are sequentially displayed for determination. In the lowermost part, an area and a judge name are displayed.

The determinations based on the three methods are sequentially and separately made, whereby determinations of the symptom level based on the three methods are separately obtained. That is, first the symptoms of the wounded person are input based on the START method, and the symptom level is determined. Then, the symptoms of the wounded person are input based on the PAT method, and the symptom level is determined. The symptom level determined based on the PAT method is overwritten on the symptom level determined based on the START method. Then, the symptoms are input based on the vital sign, and the symptom level is determined. When the symptom level determined based on the PAT method is higher (more severe) than that determined based on the vital sign, the symptom level based on the PAT method is finally adopted. In short, the symptom levels based on the previous two methods are referred to, and the highest symptom level is adopted as a final result. However, if sufficient time cannot be provided, the symptom level is sometimes displayed based on the first or second determination level.

The upper basic information display section 81 of the smartphone 14a has input fields of name, age, gender, disease name, and final evaluation. On a right side of these fields, a photograph 84 of the wounded person is displayed.

The above information is written in the known range, and the photograph, if obtained, is also input as digital data. The final evaluation is displayed after completion of evaluation. After the personal basic data of the wounded person is input to the smartphone 14a in step S501, the input personal basic data is transmitted to the triage tag 13 of the corresponding wounded person in step S502. The communication between the triage tag 13 and smartphone 14 is performed at close range and, thus, the triage tag 13 can receive the data without fail.

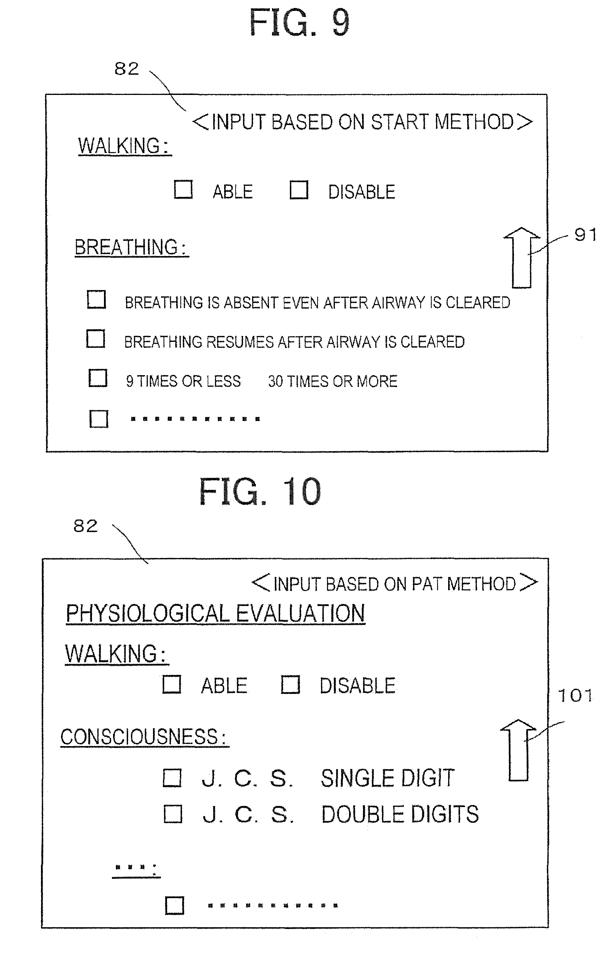

Then, first the determination based on the START method is made in accordance with the symptom of the wounded person. That is, in step S503, the wounded person is observed and diagnosed, and symptom data is input. FIG. 9 illustrates a display example of the condition input/determination display section 82 when the symptoms of the wounded person are input by the START method. Whether or not the wounded person can walk is asked, and an operator of the smartphone 14a makes selective input. Then, choices on status of breathing are displayed, and the operator makes selective input. When the screen is flicked with a finger to move the screen in a direction of an arrow 91, the succeeding screen appears.

Generally, in the determination based on the START method, whether or not the wounded person can walk, presence/absence of breathing, a breathing rate, a circulation level, and a conscious level are sequentially input. However, sometimes the symptom level is determined in the middle of the input operation, depending on the selected symptom. For example, in a state where the wounded person cannot walk and breathing is absent, when the breathing is still absent even after the airway is cleared, a level 0 (black: death) is determined. When the breathing resumes after the airway is cleared, a level 1 (red: emergency treatment) is determined.

In subsequent step S504, the symptom level based on the START method is determined. It is not always necessary for all the symptoms to be input for the determination in step S504. That is, if the symptom level is fixed by a given selected symptom in the middle of the input operation, the determination in step S504 is completed at that time. Thus, for example, when "breathing resumes after airway is cleared" is selected in the condition of breath in FIG. 9, the symptom level 1 is determined at this time.

The determination result (symptom level determined based on the START method) is transmitted, together with the determination time thereof, to the triage tag 13 and triage support server 15 in step S505. In addition to the symptom level, the input detailed data can be transmitted to the triage tag 13 and triage support server 15. This allows the detailed data contents to be analyzed in a time-series manner, and the analysis result can be used as a reference for detailed diagnosis. Details of a procedure for transmission of the determination result are illustrated in FIG. 6.

In step S601, the symptom level determined based on the START method is transmitted to the triage tag 13. The transmission of the symptom level data is repeated until the transmission is completed in step S602. After completion of the transmission, a processing flow proceeds to step S603. In step S603, the symptom data obtained as the determination result is transmitted to the triage support server 15 through the wireless LAN 11L and network 11.

In step S604, the connection detection section 27D of the smartphone 14 (14a) determines whether the determination result can be transmitted from the smartphone to triage support server 15.

The symptom level that has been transmitted to the triage support server is stored, together with the determination time thereof, in the wounded person information DB 12 for each wounded person.

When the connection detection section 27D determines that the determination result cannot be transmitted, the symptom level as the determination result is once stored in the temporary storage section in step S605.

In subsequent step S606, it is detected whether the transmission to the triage support server 15 is possible. When the condition of the network 11 or wireless LAN 11L does not still allow the symptom level to be transmitted, the processing flow returns to step S605, where the temporary storage section 28 continues the storage of the symptom level.

Then, when the transmission to the triage support server 15 becomes possible in step S606, the transmission of the symptom level is continued.

Also when the transmission to the triage support server 15 is possible in step S604, the processing flow proceeds to step S607, where the transmission of the symptom level is continued. In step S608, the symptom level is stored together with the determination time thereof to the wounded person information DB 12.

Then, the processing flow returns to step S506 of FIG. 5, where the symptom data are input to the smartphone 14a based on the PAT method.

FIG 10 illustrates an example of a screen to be displayed first for input operation based on the PAT method. In this example, whether or not the wounded person can walk and whether a conscious level (depth of disturbance of consciousness) is single digit or double digits in J.C.S are determined. The J.C.S is initial letters of "Japan Coma Scale" and indicates the Japanese standard for the depth of disturbance of consciousness. Glasgow Coma Scale (G.C.S.) is sometimes used in place of the J.C.S. Although not illustrated, when the screen is flicked in a direction of an arrow 101 to move the screen, several items, such as a breathing rate, a pulse rate, a blood pressure, a cold/wet feeing, and a body temperature, are displayed as the symptoms for further input operation.

After performing the input operation with respect to the items of the symptoms, the symptom level is determined based on the PAT method in step S507. This determination result is also transmitted, together with the determination time thereof, to the triage tag 13 and triage support server 15 in step S508. A procedure of the transmission is as illustrated in FIG. 6. That is, the transmission to be performed in step S508 is the same as that of the transmission of the symptom level obtained based on the START method except that the data to be transmitted are the symptom level determined based on the PAT method and the determination time thereof, so the descriptions thereof will be omitted.

Also in this case, first the symptom level and determination time thereof are transmitted to the triage tag 13 and then to the triage support server 15 through the wireless LAN 11L and network 11. When the symptom level and determination time thereof cannot be transmitted to the triage support server 15, they are temporarily stored in the temporary storage section 28 and are thereafter transmitted when the transmission thereof is possible. The symptom level transmitted to the triage support server 15 is stored, together with the determination time thereof, in the wounded person information DB 12 as the data of the corresponding wounded person. The latest symptom level and determination time thereof are overwritten on the symptom level based on the START method and determination time thereof (step S608).

The processing flow shifts to step S509 of FIG. 5, where the symptom data is input to the smartphone 14a based on the vital sign.

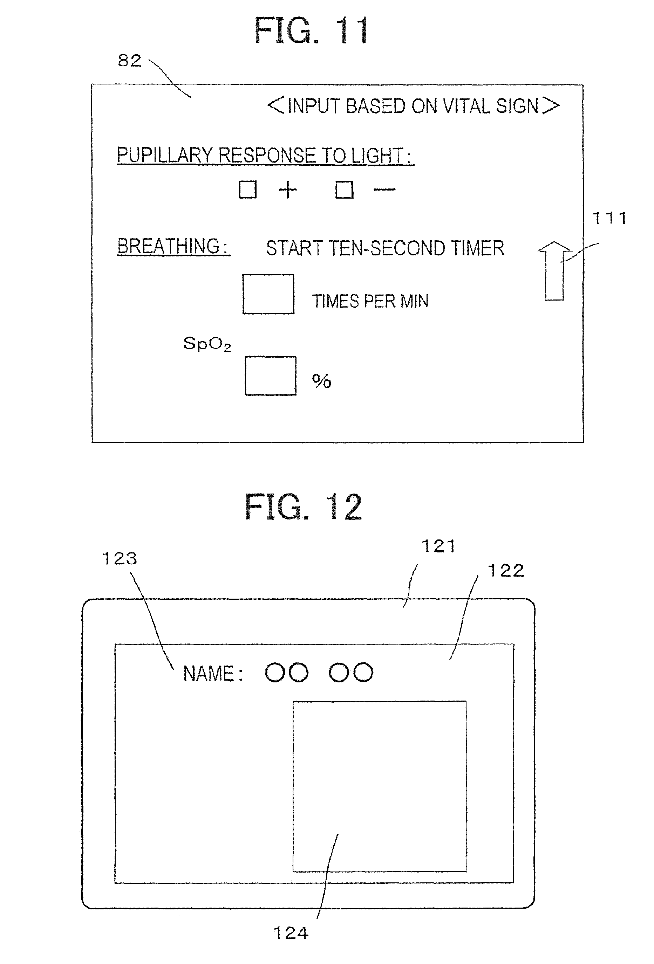

In the case of the vital sign, input items such as, pupillary response to light, breathing, body temperature, pulse rate, consciousness, and SpO2 are displayed. An example of an input screen displaying such items is illustrated in FIG. 11. The SpO2 is oxygen saturation of the blood (oxygen saturation of arterial blood). When the screen is flicked in a direction of an arrow 111 to move the screen, further input items can be displayed. Numerical values are often used in the input of vital sign, and the symptom level is determined based on whether the numerical value falls within a predetermined normal value range.

In step S510, the symptom level based on the vital sign is determined. In step S511, the symptom level determined based on the START method in step S504 and symptom level determined based on the PAT method in step S507 are referred to, and the highest one of the symptom levels based on the START method, PAT method, and vital sign is determined.

In step S512, the determined symptom level is transmitted to the triage tag 13 and triage support server 15.

A procedure of the transmission is also as illustrated in FIG. 6. That is, the symptom level of the wounded person determined based on the vital sign is transmitted to the triage tag 13 and then to the triage support server 15. At this time, when the connection detection section 27D determines that the condition of the network 11 or the like does not allow the symptom level and the like to be transmitted, the symptom level and the like are temporarily stored in the temporary storage section 28. Thereafter, when the transmission thereof is possible, the symptom level and the like are transmitted to the triage support server 15 through the network 11.

According to the procedures illustrated in FIGS. 5 and 6, the symptom level and determination time thereof determined based on the symptoms input to the smartphone 14a and the three determination methods are transmitted to the triage tag 13 and triage support server 15.

By the way, in the actual disaster site, it is not always true that all the symptom levels are successfully stored and updated in the triage support server 15. There may be a case where malfunction of network continues or where a doctor of a wounded person attached with the triage tag 13 is changed.

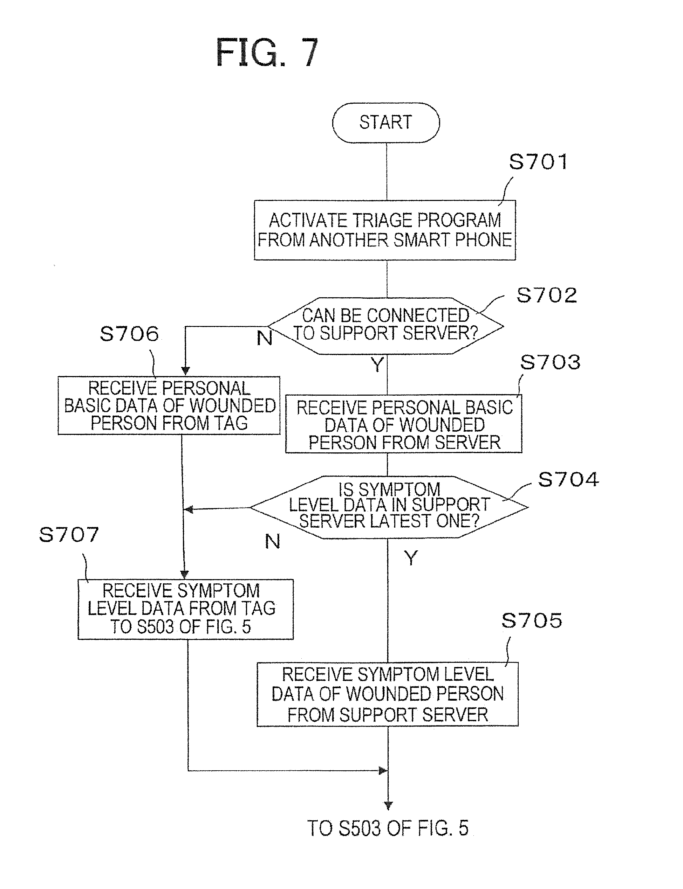

Such a case will be described based on a flowchart illustrated in FIG. 7. Assume that a doctor who owns another smartphone 14b takes over the care of the wounded person. In step S701, the triage program is activated on the smartphone 14b, and whether the smartphone 14b can be connected to the triage support server 15 is checked in step S702.

The reason that the data of the triage support server 15 is prioritized is because a download time is shorter for the triage support server 15 than that for the triage tag 13.

When the smartphone 14b can be connected to the triage support server 15, the personal basic data transmitted from the triage support server 15 is received by the smartphone 14b in step S703. In subsequent step S704, it is confirmed whether the symptom level data in the triage support server 15 is the latest one or not. This confirmation is made based on, e. g. , the determination time of the symptom level.

When the symptom level data in the triage support server 15 is the latest one, the symptom level data of the corresponding wounded person is received from the triage support server 15 in step S705.

Then, the processing flow returns to step S503 of FIG. 5, where the symptom data is input based on the START method. The subsequent input and determination operations are the same as those described above except that they are performed on the smartphone 14b. When it is clear that the input and determination operations based on the START method, PAT method, and vital sign have been made halfway, the input and determination operations can be resumed from there.

On the other hand, when the connection detection section 27D of the smartphone 14b detects that the smartphone 14b cannot be connected to the triage support server 15 in step S702, the processing flow shifts to step S706, where the personal basic data of the wounded person is received from the triage tag 13.

This is achieved by transmitting an inquiry signal from the tag transmission/reception section 26 of the smartphone 14b to the triage tag 13. In the triage tag 13, based on a control signal transmitted from the smartphone transmission/reception section 33 to the personal information storage section 31, the personal basic data is transmitted from the personal information storage section 31 to the smartphone 14b through the smartphone transmission/reception section 33.

In subsequent step S707, the symptom level data is transmitted from the symptom level storage section 32 of the triage tag 13 to the smartphone 14b. In this manner, even when the data cannot be acquired from the triage support server 15, the personal basic data and the latest symptom data of the wounded person can be stored in the smartphone 14b.

Also when it is detected in step S704 that the symptom level data of the wounded person is not the latest one in a state where the smartphone 14b is connected to the triage support server 15, the processing flow shifts to step S707, where the symptom level data is acquired from the triage tag 13. This is because there may be a case where the communication between the smartphone 14a and network is disabled during input operation of the symptom data based on the START method, PAT method, and vital sign to result in failing to update the symptom level stored in the wounded person information DB 12 in the triage support server 15 with the latest one.

After the smartphone 14b acquires the personal basic data of the wounded person and the latest symptom level data in step S705 or S707 of FIG. 7, determination of the symptom level of the wounded person is made once again in step S503 and subsequent steps.

According to the present embodiment, there can be provided a triage tag management system and the like capable of grasping the latest information such as symptoms of wounded persons to thereby adequately make an appropriate response even under a condition that the network connection state becomes unstable.

In the above embodiment, the triage tag incorporates therein the IC tag, in which all the information related to the wounded person is stored. However, the triage tag may have a display section, in addition to the IC tag, and configured to display a name and a photograph of the wounded person based on the information stored in the IC tag. A triage tag of this embodiment is illustrated in FIG. 12. That is, an electrical display section 122 is provided on a triage tag 121 so as to allow a name 123 and a photograph 124 to be displayed on the electrical display section 122. The display section 122 can be made visible or invisible. Moreover, the display section 122 may be put in an invisible state after elapse of a predetermined time from the start of a visible state. This can prolong the life of a built-in battery.

According to the tag of this embodiment, it is possible to confirm, at first sight, a face and a name of the wounded person, thereby preventing one wounded person from being mistaken for another.

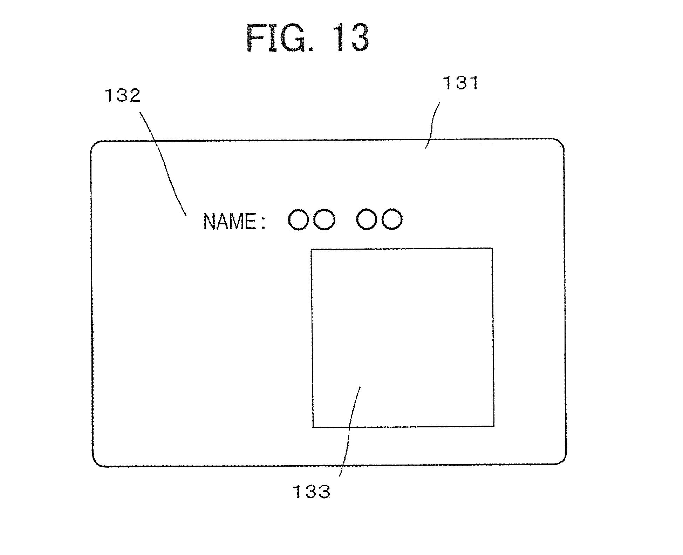

Moreover, the triage tag may have, in addition to the IC tag, a display area on which a name is printed or written, or a taken photograph is attached. FIG. 13 illustrates a triage tag 131 of this embodiment. Onto a surface of the triage tag 131, a name 132 of the wounded person is printed or written, and a photograph 133 thereof is attached. As described above, a physical display section may be provided on the triage tag 131.

According to the triage tag of this embodiment, it is possible to display basic personal information without providing the electrical display section. This prevents misidentification of the wounded persons and reduces cost.

The smartphone used in the present invention may be any type of mobile terminal as long as an image is displayed thereon and input is recognized through a contact to a screen thereof.

In the above embodiments, the smartphone, triage tag, and triage support server each have an electrical configuration. However, the present invention is not limited to this, and the smartphone, triage tag, and triage support server each may be configured to perform its operation by an internal program.

Although the preferred embodiments of the present invention have been described above, the embodiments are merely illustrative and do not limit the scope of the present invention. These novel embodiments can be practiced in other various forms, and various omissions, substitutions and changes may be made without departing from the scope of the invention. The embodiments and modifications thereof are included in the scope or spirit of the present invention and in the appended claims and their equivalents.

### REFERRENCE SIGN LIST

- 11:: Network
- 11L:: Wireless LAN
- 12:: Wounded person information database (DB)
- 13:: Triage tag
- 14, 14a, 14b:: Smartphone
- 15:: Triage support server
- 20:: Display input section
- 21:: Display controller
- 22:: Input recognition section
- 23S:: START method control/determination section
- 23P:: PAT method control/determination section
- 23V:: Vital sign control/determination section
- 24:: Symptom level decision section
- 25:: Symptom level storage section
- 26:: Tag transmission reception section
- 27:: Server transmission/reception section
- 27D, 42D:: Connection detection section
- 28, 44:: Temporary storage section
- 81:: Basic information display section
- 82:: Condition input/determination display section
- 31:: Personal information storage section
- 32:: Symptom level storage section
- 33:: Smartphone transmission/reception section
- 41:: Data search section
- 42:: Smartphone transmission/reception section
- 43:: Data write/read section

## Claims

1. A triage tag management system comprising:
a triage tag attached to a wounded person;
a smartphone that receives input of symptom data of the wounded person, determines a symptom level of the wounded person, and transmits data of the symptom level to the triage tag; and
a wounded person information database that receives the symptom level data of the wounded person from the smartphone over a network and stores the symptom level data therein for each wounded person attached with the triage tag, the smartphone including a temporary storage section that temporarily stores the symptom level data of the wounded person when the symptom level data cannot be transmitted to the wounded person information database.

2. The triage tag management system according to claim 1, wherein
the smartphone includes a symptom determination section that receives input of symptoms of the wounded person based a predetermined determination method and determines the symptom level of the wounded person.

3. The triage tag management system according to claim 2, wherein
the predetermined determination method uses a START method, a PAT method, and a vital sign, inputs the symptoms of the wounded person in this order, and determines the symptom level of the wounded person.

4. The triage tag management system according to claim 3, wherein
the symptom determination section includes a START method control/determination section that determines the symptoms of the wounded person using input based on the START method, a PAT method control/determination section that determines the symptoms of the wounded person using input based on the PAT method, and a vital sign control/determination section that determines the symptoms of the wounded person using input based on the vital sign.

5. The triage tag management system according to claim 4, wherein
the triage tag includes an electrical or physical display section.

6. A smartphone for triage tag management system, comprising:
a tag transmission/reception section that receives input of symptom data of a wounded person, determines a symptom level of the wounded person, transmits data of the symptom level to a triage tag attached to the wounded person, and receives data from the triage tag;
a server transmission/transmission section that transmits, over a network, the symptom level data of the wounded person to a triage support server including a wounded person information database that stores the symptom data for each wounded person attached with the triage tag and receives data from the wounded person information database; and
a temporary storage section that temporarily stores the symptom level data of the wounded person when the symptom level data cannot be transmitted to the wounded person information database.

7. The smartphone for triage tag management system according to claim 6, further comprising a symptom determination section that receives input of symptoms of the wounded person based on a predetermined determination method and determines the symptom level of the wounded person.

8. The smartphone for triage tag management system according to claim 7, wherein
the predetermined determination method uses a START method, a PAT method, and a vital sign, inputs the symptoms of the wounded person in this order, and determines the symptom level of the wounded person.

9. The smartphone for triage tag management system according to claim 8, wherein
the symptom determination section includes a START method control/determination section that determines the symptoms of the wounded person using input based on the START method, a PAT method control/determination section that determines the symptoms of the wounded person using input based on the PAT method, and a vital sign control/determination section that determines the symptoms of the wounded person using input based on the vital sign.

10. A triage tag management method comprising:
inputting symptom data of a wounded person to a smartphone;
determining a symptom level based on the input symptom data of the wounded person and transmitting the symptom level data to a triage tag attached to the wounded person;
transmitting, over a network, the symptom level data of the wounded person from the smartphone to a wounded person information database that stores the symptom level data therein for each wounded person attached with the triage tag; and
temporarily storing the symptom level data of the wounded person in a temporary storage section of the smartphone when the symptom level data cannot be transmitted from the smartphone to the wounded person information database.

11. The triage tag management method according to claim 10, wherein
the smartphone receives input of symptoms of the wounded person based on a predetermined determination method and determines the symptom level of the wounded person.

12. The triage tag management method according to claim 11, wherein
the predetermined determination method uses a START method, a PAT method, and a vital sign, inputs the symptoms of the wounded person in this order, and determines the symptom level of the wounded person.

13. The triage tag management method according to claim 12, wherein
the symptom determination section sequentially determines the symptom level of the wounded person using input based on the START method, input based on the PAT method, and input based on the vital sign in this order.
